# EUROPEAN PATENT APPLICATION

(11) **EP 2 862 924 A1**
(43) Date of publication of application: **22.04.2015**
(21) Application number: 13803610.8
(22) Date of filing: 13.06.2013
(51) Int. Cl.: C12M 1/34, C12Q 1/00

(54) **INTERACTION PREDICTION DEVICE, INTERACTION PREDICTION METHOD, AND PROGRAM**

(30) Priority: 13.06.2012 JP 2012134261
(71) Applicant: Okinawa Institute of Science and Technology Graduate University, Okinawa 904-0495 (JP); The Systems Biology Institute, Tokyo 108-0071 (JP)
(72) Inventor: KITANO, Hiroaki, Kunigami-gun Okinawa 904-0495 (JP); HSIN, Kun-yi,, Kunigami-gun Okinawa 904-0495 (JP); GHOSH, Samik, Tokyo 108-0071 (JP)
(74) Representative: Hoffmann Eitle
(86) International application number: PCT/JP2013/066323
(87) International publication number: WO 2013/187468

(57) **Abstract**

The present invention acquires compound structure data and candidate protein structure data on a candidate protein serving as a candidate for interaction with the compound. The present invention calculates a binding strength between the candidate protein and the compound using a docking simulation method, determines a predicted binding strength corresponding to the binding strength predicted by making a comprehensive evaluation of the binding strength, and determines a predicted protein corresponding to the candidate protein predicted to interact with the compound. The present invention calculates an interaction strength using a binding strength simulation method and determines a predicted interaction strength corresponding to the interaction strength predicted by making the comprehensive evaluation of the interaction strength.

## Description

### TECHNICAL FIELD

The present invention relates to an interaction prediction device, an interaction prediction method, and a computer program product.

### BACKGROUND ART

Conventionally, technologies for predicting biomolecular binding are disclosed.

The ligand docking system described in Non Patent Literatures 1 and 2 causes all ligand-derived rigid fragments to dock in receptor sites. Thus, the ligand docking system applies a flexible docking algorithm including fine sampling of the atomic position of the rigid fragments and successive fine adjustment of a dihedral angle of a rotatable bond to a drug design.

### CITATION LIST

### NON PATENT LITERATURE

Non Patent Literature 1: Zsoldos Z, Reid D, Simon A, Sadjad BS, Johnson AP. eHiTS: an innovative approach to the docking and scoring function problems. Curr Protein Pept Sci. 2006 Oct; 7(5): 421-35.
Non Patent Literature 2: Zsoldos Z, Reid D, Simon A, Sadjad SB, Johnson AP. eHiTS: a new fast, exhaustive flexible ligand docking system. J Mol Graph Model. 2007 Jul; 26(1): 198-212. Epub 2006 Jun 17.

### SUMMARY OF INVENTION

### PROBLEM TO BE SOLVED BY THE INVENTION

The conventional ligand docking system described in Non Patent Literatures 1 and 2 identifies a target molecule with which a candidate compound for development of a new drug mainly interacts. In many cases, however, the conventional ligand docking system recognizes only one or a few of many biomolecules with which the candidate compound interact as a target molecule. As a result, in the conventional ligand docking system, a drug development process proceeds on the assumption that the candidate compound interacts with only a target molecule determined arbitrarily in a sense. Thus, effects of the candidate compound expected by a user, such as a researcher for a drug development company, may possibly differ from actual effects. This is because a candidate compound typically interacts not with a single biomolecule but with many biomolecules at various strengths, and the obtained comprehensive effects serve as the actual effects of the candidate compound.

In view of the disadvantage described above, the present invention aims to provide an interaction prediction device, an interaction prediction method, and a computer program product that can predict which intravital protein a chemical substance, such as a compound, interacts with and how the interaction affects a living body.

### Solution to Problem

### MEANS FOR SOLVING PROBLEM

In order to attain this object, an interaction prediction device according to one aspect of the present invention is an interaction prediction device comprising a storage unit and a control unit, wherein the storage unit includes a compound structure data storage unit that stores compound structure data on a structure of a compound, and a protein structure data storage unit that stores protein structure data on a structure of a protein, and the control unit includes a compound structure data acquiring unit that acquires the compound structure data on the compound from the compound structure data storage unit or predicts and acquires the compound structure data not stored in the compound structure data storage unit using a structure prediction method, a protein structure data acquiring unit that acquires candidate protein structure data corresponding to the protein structure data on a candidate protein serving as the protein to be a candidate for interaction with the compound from the protein structure data storage unit or predicts and acquires the candidate protein structure data not stored in the protein structure data storage unit using the structure prediction method, a predicted protein determining unit that calculates a binding strength between the candidate protein and the compound using a docking simulation method based on the compound structure data acquired by the compound structure data acquiring unit and the candidate protein structure data acquired by the protein structure data acquiring unit, determines a predicted binding strength corresponding to the binding strength eventually predicted by making a comprehensive evaluation of the binding strength using any one or both of a learning method and a meta-estimation method, and determines a predicted protein corresponding to the candidate protein predicted to interact with the compound, and an interaction strength determining unit that calculates an interaction strength using a binding strength simulation method based on the compound structure data acquired by the compound structure data acquiring unit and the protein structure data on the predicted protein determined by the predicted protein determining unit and determines a predicted interaction strength corresponding to the interaction strength eventually predicted by making the comprehensive evaluation of the interaction strength using any one or both of the learning method and the meta-estimation method.

The interaction prediction device according to another aspect of the present invention is the interaction prediction device, wherein the protein structure data storage unit stores the protein structure data on the structure of the protein in association with network data on an intracellular or intravital network including position data on the position of the protein on the network, and the control unit further includes an influence predicting unit that predicts an influence of the compound on the predicted protein based on the predicted interaction strength determined by the interaction strength determining unit and the network data stored in the protein structure data storage unit.

The interaction prediction device according to still another aspect of the present invention is the interaction prediction device, wherein the storage unit further includes an intermolecular interaction data storage unit that stores intermolecular interaction data on intracellular or intravital intermolecular interaction, and any one or both of the predicted protein determining unit and the interaction strength determining unit make the comprehensive evaluation further using the intermolecular interaction data stored in the intermolecular interaction data storage unit.

The interaction prediction device according to still another aspect of the present invention is the interaction prediction device, wherein the storage unit further includes a protein structure similarity data storage unit that stores protein structure similarity data on similarity in the structure of the protein, and any one or both of the predicted protein determining unit and the interaction strength determining unit make the comprehensive evaluation further using the protein structure similarity data stored in the protein structure similarity data storage unit.

The interaction prediction device according to still another aspect of the present invention is the interaction prediction device, wherein the protein structure data acquiring unit predicts and acquires the candidate protein structure data by predicting a plurality of pieces of protein structure data using the structure prediction method and making the comprehensive evaluation of the pieces of protein structure data using any one or both of the learning method and the meta-estimation method.

The interaction prediction device according to still another aspect of the present invention is the interaction prediction device, wherein the storage unit further includes a genetic data storage unit that stores genetic data on a gene of an individual, and the protein structure data acquiring unit predicts and acquires the candidate protein structure data using the structure prediction method based on the genetic data stored in the genetic data storage unit.

An interaction prediction method according to still another aspect of the present invention is an interaction prediction method executed by an interaction prediction device including a storage unit and a control unit, wherein the storage unit includes a compound structure data storage unit that stores compound structure data on a structure of a compound, and a protein structure data storage unit that stores protein structure data on a structure of a protein, the method executed by the control unit comprising a compound structure data acquiring step of acquiring the compound structure data on the compound from the compound structure data storage unit or predicting and acquiring the compound structure data not stored in the compound structure data storage unit using a structure prediction method, a protein structure data acquiring step of acquiring candidate protein structure data corresponding to the protein structure data on a candidate protein serving as the protein to be a candidate for interaction with the compound from the protein structure data storage unit or predicting and acquiring the candidate protein structure data not stored in the protein structure data storage unit using the structure prediction method, a predicted protein determining step of calculating a binding strength between the candidate protein and the compound using a docking simulation method based on the compound structure data acquired at the compound structure data acquiring step and the candidate protein structure data acquired at the protein structure data acquiring step, determining a predicted binding strength corresponding to the binding strength eventually predicted by making a comprehensive evaluation of the binding strength using any one or both of a learning method and a meta-estimation method, and determining a predicted protein corresponding to the candidate protein predicted to interact with the compound, and an interaction strength determining step of calculating an interaction strength using a binding strength simulation method based on the compound structure data acquired at the compound structure data acquiring step and the protein structure data on the predicted protein determined at the predicted protein determining step and determining a predicted interaction strength corresponding to the interaction strength eventually predicted by making the comprehensive evaluation of the interaction strength using any one or both of the learning method and the meta-estimation method.

A computer program product according to still another aspect of the present invention is a computer program product having a non-transitory tangible computer-readable medium including programmed instructions for causing, when executed by an interaction prediction device including a storage unit including a compound structure data storage unit that stores compound structure data on a structure of a compound, and a protein structure data storage unit that stores protein structure data on a structure of a protein, and a control unit, the control unit to perform a method comprising a compound structure data acquiring step of acquiring the compound structure data on the compound from the compound structure data storage unit or predicting and acquiring the compound structure data not stored in the compound structure data storage unit using a structure prediction method, a protein structure data acquiring step of acquiring candidate protein structure data corresponding to the protein structure data on a candidate protein serving as the protein to be a candidate for interaction with the compound from the protein structure data storage unit or predicting and acquiring the candidate protein structure data not stored in the protein structure data storage unit using the structure prediction method, a predicted protein determining step of calculating a binding strength between the candidate protein and the compound using a docking simulation method based on the compound structure data acquired at the compound structure data acquiring step and the candidate protein structure data acquired at the protein structure data acquiring step, determining a predicted binding strength corresponding to the binding strength eventually predicted by making a comprehensive evaluation of the binding strength using any one or both of a learning method and a meta-estimation method, and determining a predicted protein corresponding to the candidate protein predicted to interact with the compound, and an interaction strength determining step of calculating an interaction strength using a binding strength simulation method based on the compound structure data acquired at the compound structure data acquiring step and the protein structure data on the predicted protein determined at the predicted protein determining step and determining a predicted interaction strength corresponding to the interaction strength eventually predicted by making the comprehensive evaluation of the interaction strength using any one or both of the learning method and the meta-estimation method.

### EFFECT OF THE INVENTION

The present invention acquires compound structure data on a compound or predicts and acquires compound structure data that is not stored using a structure prediction method. The present invention acquires candidate protein structure data corresponding to protein structure data on a candidate protein serving as a protein to be a candidate for interaction with the compound or predicts and acquires candidate protein structure data that is not stored using the structure prediction method. The present invention calculates a binding strength between the candidate protein and the compound using a docking simulation method based on the acquired compound structure data and the acquired candidate protein structure data. The present invention then determines a predicted binding strength corresponding to the binding strength eventually predicted by making a comprehensive evaluation of the binding strength using any one or both of a learning method and a meta-estimation method, and determines a predicted protein corresponding to the candidate protein predicted to interact with the compound. The present invention calculates an interaction strength using a binding strength simulation method based on the acquired compound structure data and the protein structure data on the determined predicted protein. The present invention then determines a predicted interaction strength corresponding to the interaction strength eventually predicted by making the comprehensive evaluation of the interaction strength using any one or both of the learning method and the meta-estimation method. Thus, the present invention can efficiently identify a biomolecule, such as a protein, with which a candidate compound interacts in a living body in development of a new drug or the like.

The present invention predicts an influence of the compound on a predicted protein based on a determined predicted interaction strength and stored network data. Thus, the present invention can significantly increase accuracy in a prediction of an effect and a side effect of the compound.

The present invention makes the comprehensive evaluation further using stored intermolecular interaction data. Thus, the present invention can make the comprehensive evaluation more accurately using the known data as an index.

The present invention makes the comprehensive evaluation further using stored protein structure similarity data. Thus, the present invention can make the comprehensive evaluation more accurately using the data of a known protein similar to the candidate protein as an index.

The present invention predicts and acquires the candidate protein structure data by predicting a plurality of pieces of protein structure data using the structure prediction method and making the comprehensive evaluation of the pieces of protein structure data using any one or both of the learning method and the meta-estimation method. Thus, the present invention can further eliminate arbitrariness from a target molecule.

The present invention predicts the candidate protein structure data using the structure prediction method based on stored genetic data. Thus, the present invention can predict a difference in the structure of proteins based on a difference in the gene sequence between individuals, thereby estimating individual differences in the influence of the candidate compound.

### BRIEF DESCRIPTION OF DRAWINGS

FIG. 1 is a flowchart of a basic principle of the present embodiment.
FIG. 2 is a block diagram of an example of a configuration of an interaction prediction device according to the present embodiment.
FIG. 3 is a flowchart of an example of processing performed by the interaction prediction device according to the present embodiment.
FIG. 4 is a schematic diagram of an example of predicted binding strength determination processing according to the present embodiment.
FIG. 5 is a schematic diagram of an example of the predicted binding strength determination processing according to the present embodiment.
FIG. 6 is a schematic diagram of an example of predicted interaction strength determination processing according to the present embodiment.
FIG. 7 is a schematic diagram of an example of the predicted interaction strength determination processing according to the present embodiment.
FIG. 8 is a schematic diagram of an example of interaction strength prediction processing according to the present embodiment.
FIG. 9 is a schematic diagram of an example of influence prediction processing according to the present embodiment.
FIG. 10 is a graph of a result obtained by calculating and predicting the binding strength between compounds and biomolecules according to the present embodiment.
FIG. 11 is a graph of a result obtained by calculating and predicting the binding strength between the compounds and the biomolecules according to the present embodiment.
FIG. 12 is a graph of a result obtained by calculating and predicting the binding strength between the compounds and the biomolecules according to the present embodiment.
FIG. 13 is a graph of an analysis result of compounds undergoing a clinical trial as an MEK inhibitor according to the present embodiment.
FIG. 14-1 is a schematic diagram obtained by color-coding an interaction network of biomolecules based on an interaction strength derived from the analysis result shown in FIG. 13.
FIG. 14-2 is a schematic diagram obtained by color-coding an interaction network of biomolecules based on the interaction strength derived from the analysis result shown in FIG. 13.
FIG. 15 is a graph of an example of calculation prediction according to the present embodiment.

### MODE(S) FOR CARRYING OUT THE INVENTION

Embodiments of an interaction prediction device, an interaction prediction method, and a computer program product according to the present invention are explained below in greater detail with reference to the accompanying drawings. The embodiments do not intend to limit the present invention.

### Outline of an embodiment of the present invention

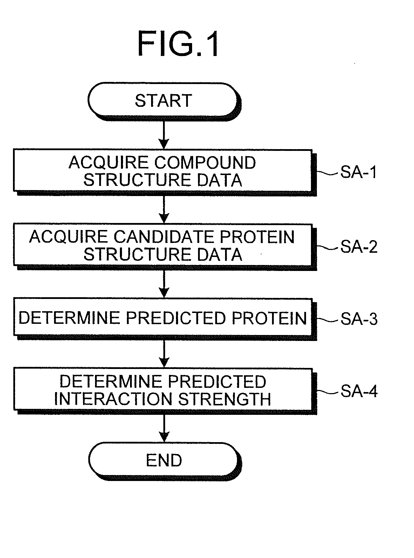

The following explains an outline of an embodiment of the present invention with reference to FIG. 1 and then explains a configuration, processing, and the like of the present embodiment in greater detail. FIG. 1 is a flowchart of a basic principle of the present embodiment. The present embodiment mainly has the following basic characteristics.

As shown in FIG. 1, a control unit of an interaction prediction device according to the present embodiment acquires compound structure data on a compound desired by a user from a storage unit. Alternatively, the control unit predicts and acquires compound structure data not stored in the storage unit using a structure prediction method (Step SA-1).

The control unit of the interaction prediction device acquires candidate protein structure data, which is protein structure data on a candidate protein serving as a protein to be a candidate for interaction with the compound, from the storage unit. Alternatively, the control unit predicts and acquires candidate protein structure data not stored in the storage unit using the structure prediction method (Step SA-2). The control unit may predict and acquire the candidate protein structure data by predicting a plurality of pieces of protein structure data using the structure prediction method and making a comprehensive evaluation of the pieces of protein structure data using any one or both of a learning method and a meta-estimation method. The control unit may predict and acquire the candidate protein structure data using the structure prediction method based on genetic data on genes of the user stored in the storage unit.

Based on the compound structure data acquired at Step SA-1 and the candidate protein structure data acquired at Step SA-2, the control unit of the interaction prediction device calculates a binding strength between the candidate protein and the compound using a docking simulation method. The control unit then determines a predicted binding strength corresponding to a binding strength eventually predicted by making a comprehensive evaluation of the binding strength using any one or both of the learning method and the meta-estimation method. Thus, the control unit determines a predicted protein corresponding to a candidate protein predicted to interact with the compound (Step SA-3). The control unit may make the comprehensive evaluation further using intermolecular interaction data stored in the storage unit. The control unit may make the comprehensive evaluation further using protein structure similarity data stored in the storage unit.

Based on the compound structure data acquired at Step SA-1 and the protein structure data on the predicted protein determined at Step SA-3, the control unit of the interaction prediction device calculates an interaction strength using a binding strength simulation method. The control unit then determines a predicted interaction strength corresponding to an interaction strength eventually predicted by making a comprehensive evaluation of the interaction strength using any one or both of the learning method and the meta-estimation method (Step SA-4) and ends the processing. The control unit may make the comprehensive evaluation further using the intermolecular interaction data stored in the storage unit. The control unit may make the comprehensive evaluation further using the protein structure similarity data stored in the storage unit.

This completes the explanation of the outline of the present embodiment.

Configuration of an interaction prediction device 100

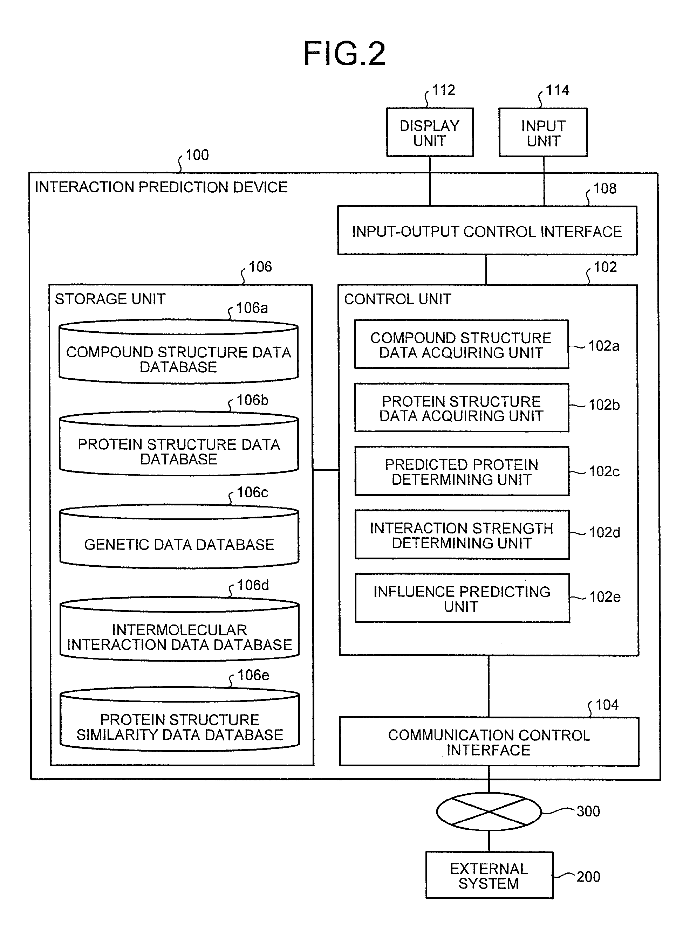

The following explains a configuration of an interaction prediction device 100 according to the present embodiment in greater detail with reference to FIG. 2. FIG. 2 is a block diagram of an example of the configuration of the interaction prediction device 100 according to the present embodiment and schematically depicts only a part relating to the present invention in the configuration. While the interaction prediction device 100 according to the present embodiment includes all the components in a single housing and performs processing alone (a stand-alone type), the embodiment is not limited thereto. The interaction prediction device 100 may have the components in separated housings and serve as a conceptual device by connecting the components via a network 300 or the like (e.g., cloud computing).

In FIG. 2, an external system 200 is interconnected with the interaction prediction device 100 via the network 300. The external system 200 may have a function to provide any one or both of an external database for any one, some, or all of protein structure data, compound structure data, genetic data, intermolecular interaction data, and protein structure similarity data, and a website that performs a user interface, for example.

The external system 200 may serve as a Web server, an ASP server, or the like. The hardware configuration of the external system 200 may include a commercially available information processor, such as a workstation and a personal computer, and auxiliary equipment thereof. Functions of the external system 200 may be carried out by a CPU, a disk drive, a memory, an input device, an output device, a communication control device, and the like in the hardware configuration of the external system 200 and by a computer program and the like for controlling these devices.

The network 300 has a function to interconnect the interaction prediction device 100 with the external system 200 and is the Internet, for example.

The interaction prediction device 100 mainly includes a control unit 102, a communication control interface 104, an input-output control interface 108, and a storage unit 106. The control unit 102 is a CPU or the like that collectively controls the entire interaction prediction device 100. The communication control interface 104 is connected to a communication device (not illustrated), such as a router, connected to a communication line or the like. The input-output control interface 108 is connected to a display unit 112 and an input unit 114. The storage unit 106 is a device that stores various types of databases, tables, and the like. These units of the interaction prediction device 100 are communicably connected via a desired communication path. The interaction prediction device 100 is communicably connected to the network 300 via a communication device, such as a router, and a wired or wireless communication line, such as a leased line.

The various types of databases and tables stored in the storage unit 106 (a compound structure data database 106a, a protein structure data database 106b, a genetic data database 106c, an intermolecular interaction data database 106d, and a protein structure similarity data database 106e) correspond to a storage unit, such as a fixed disk drive. The storage unit 106 stores various types of computer programs, tables, files, databases, and Web pages used for various types of processing, for example.

The compound structure data database 106a out of the components of the storage unit 106 stores compound structure data on a structure of a compound. The compound structure data may be stored in the compound structure data database 106a in advance. The control unit 102 of the interaction prediction device 100 may download the latest data from the external system 200 or the like via the network 300 at any one or both timings of regularly and in response to processing performed by the control unit 102. The control unit 102 then updates the compound structure data stored in the compound structure data database 106a with the latest data.

The protein structure data database 106b stores protein structure data on a structure of a protein. The protein structure data database 106b may store the protein structure data on the structure of the protein in association with network data. The network data is data on an intracellular or intravital network (e.g., an intramolecular interaction network, a signal transmission network, a metabolism network, and a genetic control network) and includes position data on the position of the protein on the network. The protein structure data may be stored in the protein structure data database 106b in advance. The control unit 102 of the interaction prediction device 100 may download the latest data from the external system 200 or the like via the network 300 at any one or both timings of regularly and in response to processing performed by the control unit 102 (e.g., at a timing when the control unit 102 requires data). The control unit 102 then updates the protein structure data stored in the protein structure data database 106b with the latest data.

The genetic data database 106c stores genetic data on genes of the user. The genetic data may include data on any one, some, or all of a base sequence, a genetic type, a genotype, a phenotype, and an annotation. The genetic data may be stored in the genetic data database 106c in advance. The control unit 102 of the interaction prediction device 100 may download the latest data from the external system 200 or the like via the network 300 at any one or both timings of regularly and in response to processing performed by the control unit 102. The control unit 102 then updates the genetic data stored in the genetic data database 106c with the latest data.

The intermolecular interaction data database 106d stores intermolecular interaction data on intracellular or intravital intermolecular interaction. The intermolecular interaction data may be stored in the intermolecular interaction data database 106d in advance. The control unit 102 of the interaction prediction device 100 may download the latest data from the external system 200 or the like via the network 300 at any one or both timings of regularly and in response to processing performed by the control unit 102. The control unit 102 then updates the intermolecular interaction data stored in the intermolecular interaction data database 106d with the latest data.

The protein structure similarity data database 106e stores protein structure similarity data on similarity in a structure of a protein. The protein structure similarity data may include data on a protein structure similarity network (PSIN). The protein structure similarity data may be stored in the protein structure similarity data database 106e in advance. The control unit 102 of the interaction prediction device 100 may download the latest data from the external system 200 or the like via the network 300 at any one or both timings of regularly and in response to processing performed by the control unit 102. The control unit 102 then updates the protein structure similarity data stored in the protein structure similarity data database 106e with the latest data.

In FIG. 2, the communication control interface 104 controls communications between the interaction prediction device 100 and the network 300 (or the communication device, such as a router). In other words, the communication control interface 104 has a function to transmit and receive data to and from the external system 200, other terminals, and the like via the communication line.

In FIG. 2, the input-output control interface 108 controls the display unit 112 and the input unit 114.

The display unit 112 may be a display unit (e.g., a display, a monitor, and a touch panel including liquid crystals or organic EL) that displays a display screen, such as an application. The input unit 114 may be a key input unit, a touch panel, a control pad (e.g., a touch pad and a game pad), a mouse, a keyboard, or a microphone, for example.

In FIG. 2, the control unit 102 includes an internal memory that stores a control program such as an operating system (OS), a computer program specifying various types of processing procedures, and required data. The control unit 102 performs information processing to perform various types of processing based on these computer programs. The control unit 102 functionally and conceptually includes a compound structure data acquiring unit 102a, a protein structure data acquiring unit 102b, a predicted protein determining unit 102c, an interaction strength determining unit 102d, and an influence predicting unit 102e.

The compound structure data acquiring unit 102a acquires compound structure data on a compound from the compound structure data database 106a. Alternatively, the compound structure data acquiring unit 102a predicts and acquires compound structure data not stored in the compound structure data database 106a using the structure prediction method.

The protein structure data acquiring unit 102b acquires candidate protein structure data corresponding to protein structure data on a candidate protein serving as a protein to be a candidate for interaction with the compound from the protein structure data database 106b. Alternatively, the protein structure data acquiring unit 102b predicts and acquires candidate protein structure data not stored in the protein structure data database 106b using the structure prediction method. The protein structure data acquiring unit 102b may predict and acquire the candidate protein structure data by predicting a plurality of pieces of protein structure data using the structure prediction method and making a comprehensive evaluation of the pieces of protein structure data using any one or both of a learning method and a meta-estimation method. The protein structure data acquiring unit 102b may predict and acquire the candidate protein structure data using the structure prediction method based on genetic data stored in the genetic data database 106c.

Based on the compound structure data acquired by the compound structure data acquiring unit 102a and the candidate protein structure data acquired by the protein structure data acquiring unit 102b, the predicted protein determining unit 102c calculates a binding strength between the candidate protein and the compound using the docking simulation method. The predicted protein determining unit 102c then determines a predicted binding strength corresponding to a binding strength eventually predicted by making a comprehensive evaluation of the binding strength using any one or both of the learning method and the meta-estimation method. Thus, the predicted protein determining unit 102c determines a predicted protein corresponding to a candidate protein predicted to interact with the compound. The predicted protein determining unit 102c may make the comprehensive evaluation further using intermolecular interaction data stored in the intermolecular interaction data database 106d. The predicted protein determining unit 102c may make the comprehensive evaluation further using protein structure similarity data stored in the protein structure similarity data database 106e.

Based on the compound structure data acquired by the compound structure data acquiring unit 102a and the protein structure data on the predicted protein determined by the predicted protein determining unit 102c, the interaction strength determining unit 102d calculates an interaction strength using the binding strength simulation method. The interaction strength determining unit 102d then determines a predicted interaction strength corresponding to an interaction strength eventually predicted by making a comprehensive evaluation of the interaction strength using any one or both of the learning method and the meta-estimation method. The interaction strength determining unit 102d may make the comprehensive evaluation further using intermolecular interaction data stored in the intermolecular interaction data database 106d. The interaction strength determining unit 102d may make the comprehensive evaluation further using protein structure similarity data stored in the protein structure similarity data database 106e.

Based on the predicted interaction strength determined by the interaction strength determining unit 102d and network data stored in the protein structure data database 106b, the influence predicting unit 102e predicts an influence of the compound on the predicted protein. The influence may be an effect (e.g., an active effect and an inhibitory effect). The influence of the compound on the protein may be activation or inactivation of the protein caused by the compound, for example.

This completes the explanation of an example of the configuration of the interaction prediction device 100 according to the present embodiment.

Processing of the interaction prediction device 100

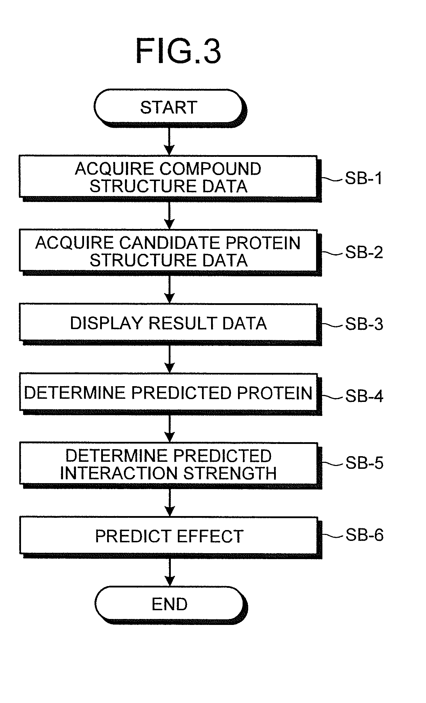

The following explains processing performed by the interaction prediction device 100 having this configuration according to the present embodiment in greater detail with reference to FIGS. 3 to 9. FIG. 3 is a flowchart of an example of processing performed by the interaction prediction device 100 according to the present embodiment.

As shown in FIG. 3, when the user develops a new drug or the like, the compound structure data acquiring unit 102a acquires compound structure data (molecular structure data) on a structure of a candidate compound serving as a compound to be a candidate of the new drug from the compound structure data database 106a. Alternatively, the compound structure data acquiring unit 102a predicts and acquires compound structure data not stored in the compound structure data database 106a using the structure prediction method (Step SB-1). The compound structure data may be input by the user through the input unit 114 and stored in the compound structure data database 106a in advance or when the processing is performed.

The structure prediction method may be any one or both of a method based on a template (template-based modeling) for estimating a structure of a protein with an unknown structure from a structure of a protein with a known structure and a method with no template (template-free modeling) for estimating a structure of a protein with an unknown structure from an amino acid sequence, which are widely used for structure prediction. Various types of methods based on a template may be used, including homology modeling and a method based on fold recognition. The structure prediction method may be a fragment assembly method. The fragment assembly method is a method for predicting a structure of a protein with an unknown structure by searching for similarity between a part of an amino acid sequence of the protein with an unknown structure and an amino acid sequence of a protein with a known structure, predicting a structure of a part of the protein with an unknown structure based on the search result, and combining a plurality of predictions. The structure prediction method may be a method of making a structure prediction of a protein as a game and acquiring a structure of a protein with an unknown structure (e.g., acquiring it via the network 300) predicted by the external system 200 (e.g., predicted by many third parties (external users) with the external system 200). The structure prediction method may be carried out by simultaneously using these methods in parallel within a possible and reasonable range. Based on the estimation results of these methods, a comprehensive evaluation is made, thereby predicting the structure of the protein with an unknown structure.

The protein structure data acquiring unit 102b acquires candidate protein structure data corresponding to protein structure data on a candidate protein serving as a protein to be a candidate for interaction with the compound from the protein structure data database 106b. Alternatively, the protein structure data acquiring unit 102b predicts and acquires candidate protein structure data not stored in the protein structure data database 106b using the structure prediction method (Step SB-2). The protein structure data acquiring unit 102b may predict and acquire the candidate protein structure data by predicting a plurality of pieces of protein structure data using the structure prediction method and making a comprehensive evaluation of the pieces of protein structure data using any one or both of the learning method and the meta-estimation method. The protein structure data acquiring unit 102b may predict and acquire the candidate protein structure data using the structure prediction method based on genetic data (personal genome data) on genes of the user stored in the genetic data database 106c. This mechanism can predict the candidate protein structure data considering that a difference in the gene sequence between individuals may possibly affect the structure of the protein and change interaction with the candidate compound, thereby changing the influence of the candidate compound. The genetic data may be input by the user through the input unit 114 and stored in the genetic data database 106c in advance or when the processing is performed.

The protein structure data acquiring unit 102b may specify one or a plurality of networks desired by the user (e.g., relating to a biological effect desired to know by the user) and specify the candidate protein from a part or all of the proteins on the networks. The protein structure data acquiring unit 102b, for example, may specify a structure of each protein on an intracellular or intravital network (e.g., an intramolecular interaction network, a signal transmission network, a metabolism network, and a genetic control network) and acquire the candidate protein structure data from the protein structure data database 106b. To predict which protein interacts with a certain compound, a candidate protein may be specified using a list of many proteins. By specifying the networks as described above, it is possible to prevent a large amount of calculation time from being spent for a protein having no relation with the focused biological influence and prevent a required protein from being absent from the list. The data on the networks may be input by the user through the input unit 114 and stored in the protein structure data database 106b in advance or when the processing is performed.

If no protein structure data is stored in the protein structure data database 106b, the protein structure data acquiring unit 102b may predict the candidate protein structure data by various types of calculation methods, that is, a structure prediction method based on molecular dynamics and the like or a method using a protein similarity network. The protein structure data acquiring unit 102b may introduce a meta-estimation system that makes a final estimation based on a plurality of different types of estimations. The meta-estimation system may use a primary sequence and a structure of a protein with a known structure and estimation results of respective estimation methods. Thus, the meta-estimation system may predict a structure of a protein with an unknown structure derived as the optimum estimation using a learning method, such as a neutral network and a support vector machine. Because it is important for the learning method to predict the structure of the protein accurately, especially to predict a structure of a site relating to interaction with the compound more accurately, the item may be weighted in the learning. If the genetic data on the user is available, the protein structure data acquiring unit 102b may analyze a coding region of each protein based on the genetic data and determine whether the structure or the like of the protein is changed based on known data. If such data is unknown, the protein structure data acquiring unit 102b may predict the candidate protein structure data by estimating an influence (e.g., whether the structure of the protein is changed) using the various types of calculation methods and considering the influence.

In the comprehensive evaluation according to the present embodiment, a structure of a protein may be estimated by carrying out in advance a plurality of structure prediction methods (estimation methods) incorporated in the present system on a plurality of proteins with a known structure. In the comprehensive evaluation, a learning method, such as the neutral network and the support vector machine, may be used to learn information indicating which evaluation method has higher evaluation accuracy on a structure of a protein having certain characteristics and on a partial structure of a certain protein. In the comprehensive evaluation, these learning results may be used to estimate a structure of a protein with an unknown structure. By performing weighting of a certainty factor on the estimation results obtained by the structure prediction methods, the candidate protein structure data may be predicted and acquired. In other words, in the comprehensive evaluation according to the present embodiment, each structure prediction method uses its characteristics that in what kind of case or on what kind of portion the method can make a highly accurate estimation, for example. If a majority vote or the like is simply used, the result varies depending on the selection of the evaluation method to be used. In the comprehensive evaluation according to the present embodiment, a predetermined learning method is used for the estimation results obtained by the structure prediction methods, thereby preventing such a bias.

Based on the compound structure data acquired by the compound structure data acquiring unit 102a and the candidate protein structure data acquired by the protein structure data acquiring unit 102b, the predicted protein determining unit 102c calculates the binding strength between the candidate protein and the compound using the docking simulation method. The predicted protein determining unit 102c then determines a predicted binding strength corresponding to a binding strength eventually predicted by making a comprehensive evaluation of the binding strength using any one or both of the learning method and the meta-estimation method. Thus, the predicted protein determining unit 102c displays the result data on the candidate protein and the predicted binding strength on the display unit 112 in a manner selectable by the user through the input unit 114 (Step SB-3). The predicted protein determining unit 102c may make the comprehensive evaluation further using intermolecular interaction data stored in the intermolecular interaction data database 106d. The intermolecular interaction data may be input by the user through the input unit 114 and stored in the intermolecular interaction data database 106d in advance or when the processing is performed. The predicted protein determining unit 102c may make the comprehensive evaluation further using protein structure similarity data stored in the protein structure similarity data database 106e. The protein structure similarity data may be input by the user through the input unit 114 and stored in the protein structure similarity data database 106e in advance or when the processing is performed.

In other words, the predicted protein determining unit 102c may run a docking simulation of each candidate protein with a series of candidate compounds to calculate the binding strength. The predicted protein determining unit 102c may run the docking simulation using a plurality of pieces of docking simulation software to determine a final predicted binding strength by evaluating the results not individually but comprehensively. A difference in methodologies employed in the respective pieces of docking simulation software leads to a bias in the prediction accuracy. To address this, the predicted protein determining unit 102c may evaluate output tendencies (inclinations) of the respective pieces of software, thereby using a combination of the optimum prediction results. At this time, the predicted protein determining unit 102c may use various types of learning methods, such as the neutral network and the support vector machine. In other words, the predicted protein determining unit 102c may use a learning method of preparing a plurality of combinations in which experimentally correct values are known, running a simulation by each method, and comparing the result and an actual experimental value. At this time, the predicted protein determining unit 102c may receive the data on the structures of the compound and the protein, the estimation results of the respective pieces of simulation software, and the like, and use a value obtained in an actual experiment as teacher data (teacher signal).

The predicted protein determining unit 102c may use the results obtained by the learning for the meta-estimation system, thereby making an estimation on binding between a compound and a protein having no measured value. In terms of the learning, to predict the data on the protein and the compound or the interaction between proteins, grouping is performed based on the data on a plurality of proteins relating thereto, and then the learning is performed in each group. This makes it possible to increase the prediction accuracy provided by the meta-estimation system that uses these results. If some biomolecules (proteins) having a similar structure are known to interact with the candidate compound, the predicted protein determining unit 102c may use such data to make an estimation. The PSIN or the like may be used to search for biomolecules having a similar structure. The predicted protein determining unit 102c may display all of the results of respective prediction modules and the results of the meta-estimation system on the display unit 112, thereby enabling the user to determine which result to use.

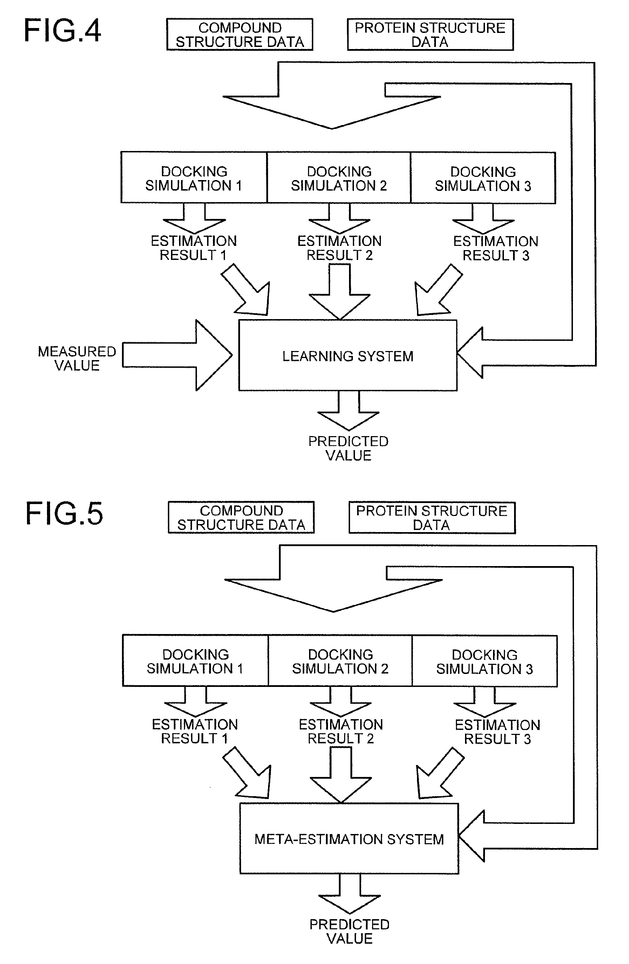

The following explains an example of predicted binding strength determination processing according to the present embodiment with reference to FIGS. 4 and 5. FIGS. 4 and 5 are schematic diagrams of an example of the predicted binding strength determination processing according to the present embodiment.

As shown in FIG. 4, the predicted protein determining unit 102c derives an estimation result 1, an estimation result 2, and an estimation result 3 of the binding strength between the candidate protein and the candidate compound using docking simulation methods of a docking simulation 1, a docking simulation 2, and a docking simulation 3, respectively, based on the compound structure data and the protein structure data (candidate protein structure data). The predicted protein determining unit 102c then determines a predicted value (predicted binding strength) corresponding to a binding strength eventually predicted by making a comprehensive evaluation of the estimation result 1, the estimation result 2, and the estimation result 3 using a learning method carried out by a learning system that uses a measured value of the binding strength as teacher data.

As shown in FIG. 5, the predicted protein determining unit 102c derives the estimation result 1, the estimation result 2, and the estimation result 3 of the binding strength between the candidate protein and the candidate compound using the docking simulation methods of the docking simulation 1, the docking simulation 2, and the docking simulation 3, respectively, based on the compound structure data and the protein structure data (candidate protein structure data). The predicted protein determining unit 102c then determines a predicted value (predicted binding strength) corresponding to a binding strength eventually predicted by making a comprehensive evaluation of the estimation result 1, the estimation result 2, and the estimation result 3 using the meta-estimation method carried out by the meta-estimation system.

Referring back to FIG. 3, if the user selects result data through the input unit 114, the predicted protein determining unit 102c determines the candidate protein predicted to interact with the candidate compound as a predicted protein based on the result data selected by the user (Step SB-4).

Based on the compound structure data acquired by the compound structure data acquiring unit 102a and the protein structure data on the predicted protein determined by the predicted protein determining unit 102c, the interaction strength determining unit 102d calculates an interaction strength using the binding strength simulation method. The interaction strength determining unit 102d then determines a predicted interaction strength corresponding to an interaction strength eventually predicted by making a comprehensive evaluation of the interaction strength using any one or both of the learning method and the meta-estimation method (Step SB-5). The interaction strength determining unit 102d may make the comprehensive evaluation further using intermolecular interaction data stored in the intermolecular interaction data database 106d. The interaction strength determining unit 102d may make the comprehensive evaluation further using protein structure similarity data stored in the protein structure similarity data database 106e. In other words, the interaction strength determining unit 102d may make an interaction strength prediction on combinations of compounds and proteins predicted to interact with each other. The interaction strength determining unit 102d may use a learning method based on results of a plurality of estimation methods and a measured value.

The binding strength simulation method (binding strength estimation method) according to the present embodiment may be an estimation method that uses a scoring function. The scoring function (e.g., X-CSCORE) may be an equation having any one, some, or all of van der Waals interaction between a compound and a protein, hydrogen bonding, an effect of structure distortion, and a hydrophobic effect as variables and solved to estimate a binding strength. A large number of such scoring functions are available, and a combination that achieves a highly accurate evaluation varies depending on the scoring functions. If a majority vote or the like is simply used, the result varies depending on the selection of the scoring function to be used. In the binding strength simulation method according to the present embodiment, a predetermined learning method may be used for binding strengths estimated by a plurality of scoring functions, thereby preventing such a bias.

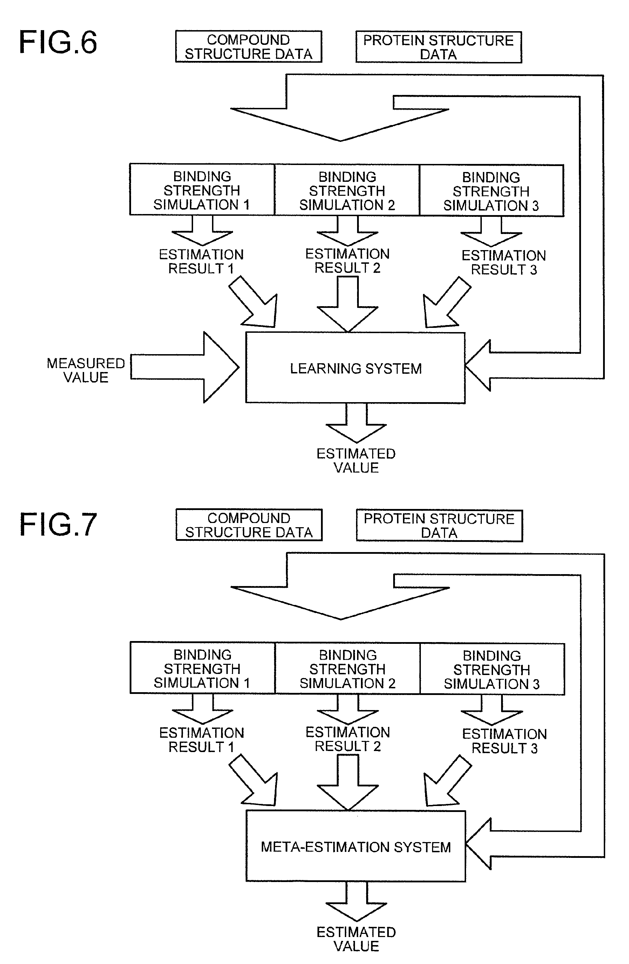

The following explains an example of predicted interaction strength determination processing according to the present embodiment with reference to FIGS. 6 and 7. FIGS. 6 and 7 are schematic diagrams of an example of the predicted interaction strength determination processing according to the present embodiment.

As shown in FIG. 6, the interaction strength determining unit 102d derives an estimation result 1, an estimation result 2, and an estimation result 3 of the interaction strength using binding strength simulation methods of a binding strength simulation 1, a binding strength simulation 2, and a binding strength simulation 3, respectively, based on the compound structure data and the protein structure data. The interaction strength determining unit 102d then determines an estimated value (predicted interaction strength) corresponding to an interaction strength eventually predicted by making a comprehensive evaluation of the estimation result 1, the estimation result 2, and the estimation result 3 using a learning method carried out by a learning system that uses a measured value of the interaction strength as teacher data.

As shown in FIG. 7, the interaction strength determining unit 102d derives the estimation result 1, the estimation result 2, and the estimation result 3 of the interaction strength using the binding strength simulation methods of the binding strength simulation 1, the binding strength simulation 2, and the binding strength simulation 3, respectively, based on the compound structure data and the protein structure data. The interaction strength determining unit 102d then determines an estimated value (predicted interaction strength) corresponding to an interaction strength eventually predicted by making a comprehensive evaluation of the estimation result 1, the estimation result 2, and the estimation result 3 using the meta-estimation method carried out by the meta-estimation system.

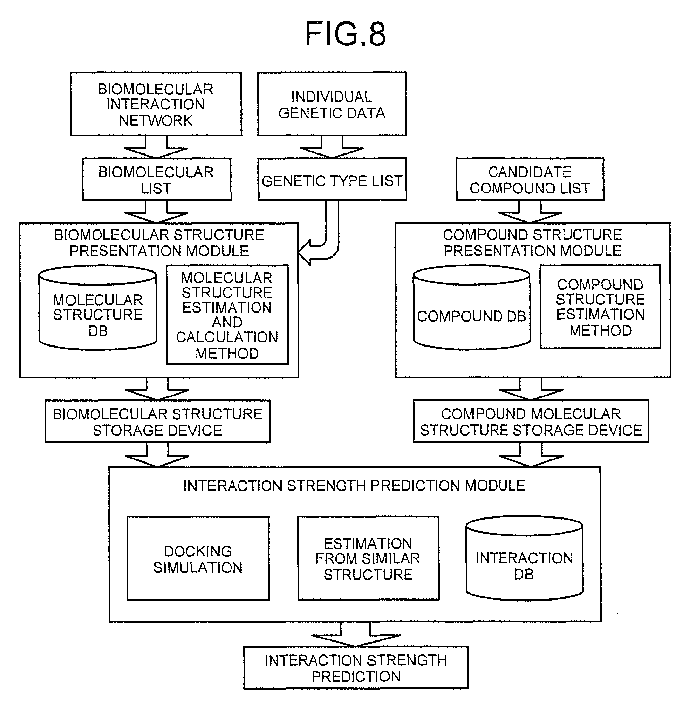

The following explains an example of interaction strength prediction processing according to the present embodiment with reference to FIG. 8. FIG. 8 is a schematic diagram of an example of the interaction strength prediction processing according to the present embodiment.

As shown in FIG. 8, if the user inputs a candidate compound list of candidate compounds serving as a compound to be a candidate for a new drug through the input unit 114, a compound structure presentation module (compound structure data acquiring unit 102a) acquires compound molecular structure data (compound structure data) on the structure of the candidate compound from a compound DB (compound structure data database 106a). Alternatively, the compound structure presentation module predicts and acquires compound molecular structure data not stored in the compound DB using a compound structure estimation method (structure prediction method). The compound structure presentation module then stores the compound molecular structure data in a compound molecular structure storage device (e.g., a memory, such as a RAM).

A biomolecular structure presentation module (protein structure data acquiring unit 102b) acquires a biomolecular list belonging to a biomolecular interaction network relating to a biological effect desired to know by the user. The biomolecular structure presentation module acquires biomolecular structure data (candidate protein structure data) corresponding to protein structure data on a candidate protein serving as a protein to be a candidate for interaction with the candidate compound and included in the biomolecular list from a molecular structure DB (protein structure data database 106b). Alternatively, if the individual genetic data on the user is available, the biomolecular structure presentation module acquires a genetic type list from the individual genetic data. The biomolecular structure presentation module predicts and acquires biomolecular structure data not stored in the protein structure data database 106b using a molecular structure estimation and calculation method (structure prediction method) while considering an influence of the structure of genes included in the genetic type list changing the structure of the protein, for example. The biomolecular structure presentation module then stores the biomolecular structure data in a biomolecular structure storage device (e.g., a memory, such as a RAM).

Based on the compound molecular structure data stored in the compound molecular structure storage device and the biomolecular structure data stored in the biomolecular structure storage device, an interaction strength prediction module (predicted protein determining unit 102c) calculates the binding strength between the candidate protein and the compound using the docking simulation method. The interaction strength prediction module then determines a predicted binding strength corresponding to a binding strength eventually predicted by making a comprehensive evaluation of the binding strength using any one or both of the learning method and the meta-estimation method. Thus, the interaction strength prediction module determines a predicted protein corresponding to a candidate protein predicted to interact with the compound.

Based on the compound molecular structure data stored in the compound molecular structure storage device and the protein structure data on the predicted protein determined by the interaction strength prediction module, the interaction strength prediction module (interaction strength determining unit 102d) calculates an interaction strength using the binding strength simulation method. The interaction strength prediction module eventually predicts an interaction strength (predicted interaction strength) by making a comprehensive evaluation of the interaction strength using the following methods. The methods are the meta-estimation method, an estimation from a similar structure based on the protein structure similarity data stored in the protein structure similarity data database 106e, and a learning method that uses intermolecular interaction data stored in an interaction DB (intermolecular interaction data database 106d) as teacher data.

Referring back to FIG. 3, based on the predicted interaction strength determined by the interaction strength determining unit 102d and network data stored in the protein structure data database 106b, the influence predicting unit 102e predicts an active effect or an inhibitory effect of the candidate compound on the predicted protein (Step SB-6) and ends the processing.

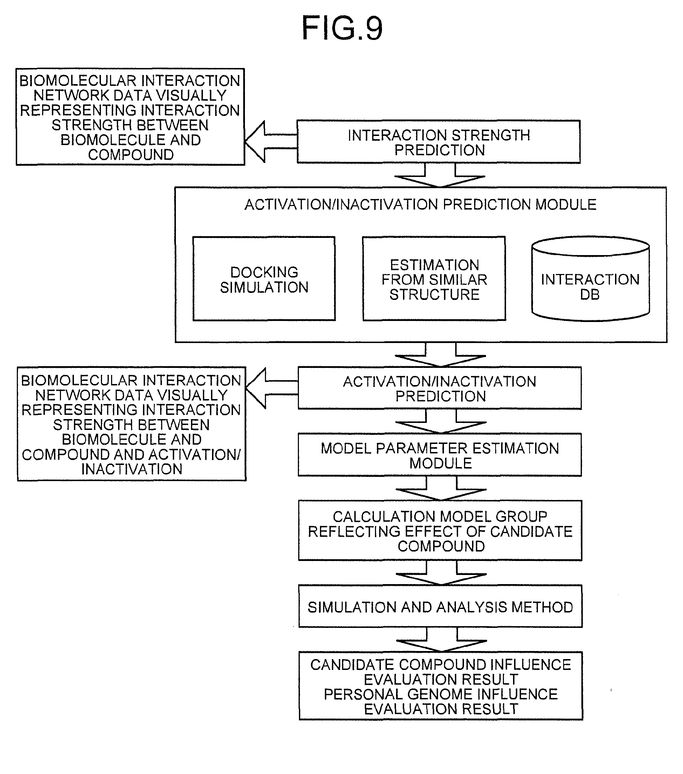

The following explains an example of influence prediction processing according to the present embodiment with reference to FIG. 9. FIG. 9 is a schematic diagram of an example of the influence prediction processing according to the present embodiment.

As shown in FIG. 9, an activation/inactivation prediction module (influence predicting unit 102e) predicts activation or inactivation caused by the candidate compound on the predicted protein based on the predicted interaction strength determined by the interaction strength determining unit 102d and network data on an intravital network including position data on the position of a biomolecule (protein) on the network stored in the protein structure data database 106b. The activation/inactivation prediction module makes the prediction using the docking simulation method, an estimation from a similar structure based on the protein structure similarity data stored in the protein structure similarity data database 106e, and a learning method that uses the intermolecular interaction data stored in the interaction DB (intermolecular interaction data database 106d) as teacher data. The activation/inactivation prediction module may indicate which protein relatively changes to an active direction or an inhibitory direction with respect to a reference standard by qualitatively propagating a direction of change on a network model and whether the result can be changed by a quantitative analysis.

In other words, the activation/inactivation prediction module sets a marker of (-) for an inhibitory property and (+) for an active property from an interaction portion of the candidate compound and propagates the markers on the network model. If an inhibitory effect propagates while maintaining the inhibitory property in the destination, for example, the activation/inactivation prediction module retains (-) and puts the mark (-) on each protein on the network model. If the propagated inhibitory property changes to the active property, the activation/inactivation prediction module replaces the mark with (+) and puts the mark (+) on each subsequent protein. After the propagation, the activation/inactivation prediction module checks which mark is assigned to a node on the network model representing each protein. The activation/inactivation prediction module may predict that a protein only with (-) assigned is inhibited and a protein only with (+) assigned is activated. As shown in FIG. 9, the activation/inactivation prediction module may further provide the user with biomolecular interaction network data (e.g., a biomolecular interaction network diagram) visually representing the interaction strength between the biomolecule and the compound (the data may be displayed on the display unit 112, for example). As shown in FIG. 9, the activation/inactivation prediction module may further provide the user with biomolecular interaction network data (e.g., a biomolecular interaction network diagram) visually representing the interaction strength between the biomolecule and the compound and activation/inactivation (the data may be displayed on the display unit 112, for example).

If predictions of activation and inactivation of the predicted protein are mixed, a model parameter estimation module (influence predicting unit 102e) needs to quantitatively analyze whether the protein is activated or inactivated. The model parameter estimation module uses a calculation model group reflecting a predicted effect (activation or inactivation) of the candidate compound on the predicted protein, thereby dynamically analyzing the intravital network. The model parameter estimation module may predict what kind of influence the candidate compound exerts on a living body with a simulation and an analysis method using any one or both of another module and known experimental data and acquire the prediction as a candidate compound influence evaluation result. The model parameter estimation module compares a model assuming a protein serving as a standard in the calculation, a model assuming a protein incorporating a change caused by a genetic type based on individual genetic data, and a model reflecting a difference in proteins caused by a plurality of pieces of individual genetic data. Thus, the model parameter estimation module may predict a difference between the individuals in the effect of the candidate compound on the predicted protein and acquire the prediction as a personal genome influence evaluation result. The method according to the present embodiment can also be used to predict toxicity of the candidate compound by specifying a target network and proteins included therein. The method according to the present embodiment can also be used to check an effect of the candidate compound on diseases other than the initially assumed disease by including networks other than the network relating to the disease initially assumed for the candidate compound as a calculation object.

The present method may be applied to prediction of interaction between proteins. The present method may also be applied to the use of a chemical substance for a plant aimed at achieving recovery from a lesion, increased productivity, or improved stress tolerance, for example.

This completes the explanation of an example of the processing of the interaction prediction device 100 according to the present embodiment.

### EXAMPLES

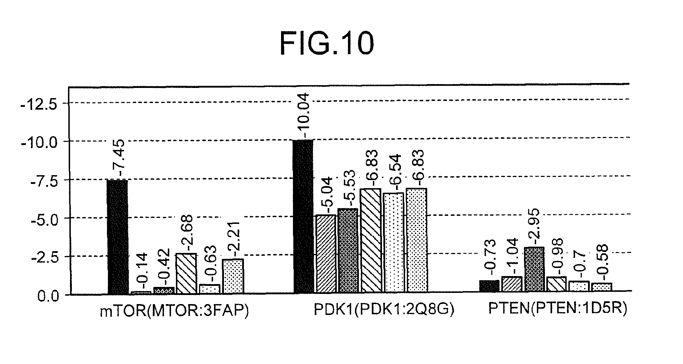

The following explains examples in which the interaction prediction method according to the present embodiment is applied to a series of candidate compounds, thereby predicting binding strengths between the series of candidate compounds and a series of biomolecules (proteins) with reference to FIGS. 10 to 15.

FIGS. 10 to 12 are graphs of results obtained by calculating and predicting the binding strength between five types of compounds (AMP, ATP, Lapatinib, Sunitinib, and Tiliroside) and three types of biomolecules (mTOR, PDK1, and PTEN) using the docking simulation method according to the present embodiment. As shown in FIGS. 10 to 12, the score represented by the leftmost bar of each biomolecule indicates the binding value (binding strength) between the biomolecule and a native ligand. The other scores of each biomolecule indicate the binding strength between the biomolecule and AMP, ATP, Lapatinib, Sunitinib, and Tiliroside in order from the left. In other words, FIGS. 10 to 12 depict an output when the user selects the five types of compounds, determines to analyze an mTOR signal transduction system, and determines not to analyze all the proteins in the transduction system but to display only the prediction for mTOR, PDK1, and PTEN among the proteins, for example. A relative value to the native ligand may be used as a reference of the binding strength between each biomolecule and each compound. Alternatively, a relative difference with the native ligand may be used as a relative binding strength based on a separately defined function.

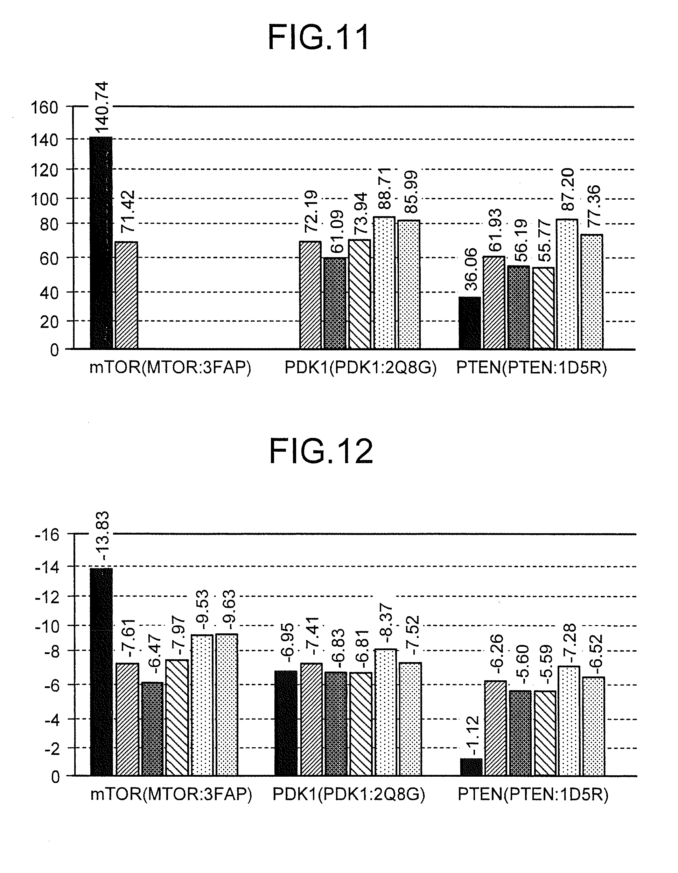

Specifically, FIG. 10 depicts an estimation result of a docking simulation eHITS when only eHITS is used in the present embodiment, that is, a calculation prediction result of the binding strengths between the five types of compounds and the three types of biomolecules. FIG. 11 depicts an estimation result of a docking simulation GOLD when only GOLD is used in the present embodiment. FIG. 12 depicts an estimation result of a docking simulation MOE when only MOE is used in the present embodiment. As described above, the present embodiment may have a function to provide the user not only with a comprehensive evaluation result of a plurality of results and an exhaustive analysis on the entire network but also with a result obtained by a specific method selected by the user while focusing on a specific molecule. As shown in FIGS. 10 to 12, however, a single method (docking simulation) is often insufficient. In other words, the estimation result may possibly significantly vary depending on the methods as shown in FIGS. 10 to 12. Specifically, in the estimation result obtained by eHITS shown in FIG. 10, the binding strength between mTOR and Lapatinib is obviously higher than that between mTOR and Sunitinib. In the estimation result obtained by MOE shown in FIG. 12, the binding strength between mTOR and Sunitinib is slightly higher than that between mTOR and Lapatinib. In the estimation result obtained by GOLD shown in FIG. 11, the binding strength between mTOR and Lapatinib and that between mTOR and Sunitinib are not estimated. As described above, an estimation with a single method may possibly face an inclination of each estimation method and its technological limit. To address this, the present embodiment may determine an eventually predicted binding strength by making a comprehensive evaluation of the binding strength using any one or both of the learning method and the meta-estimation method.

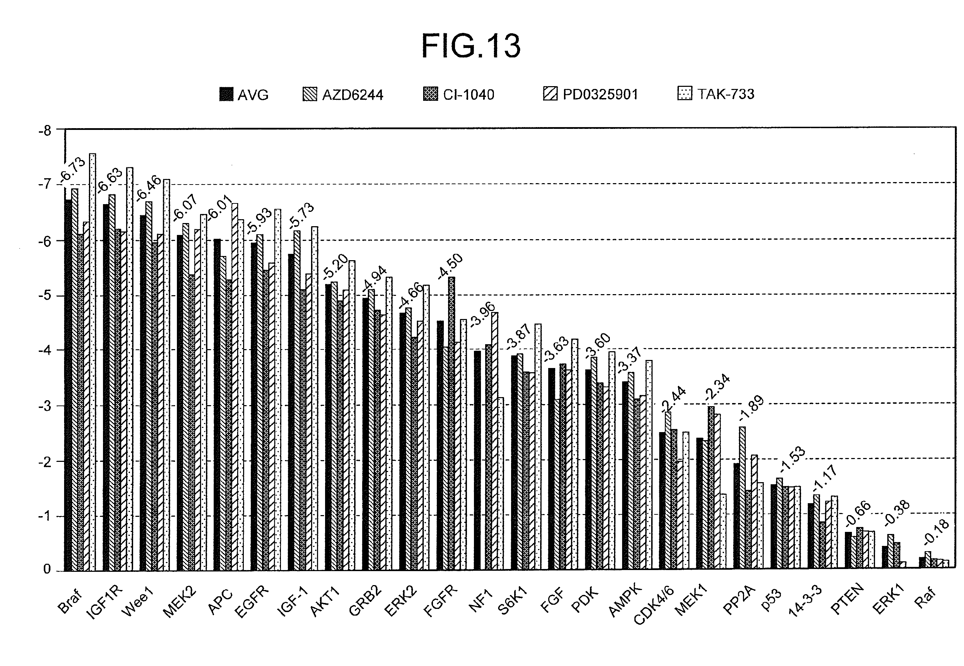

FIG. 13 depicts an analysis result of compounds of AZD6244, CI-1040, PD0325901, and TAK-733 undergoing a clinical trial as an MEK inhibitor according to the present embodiment. As shown in FIG. 13, all the compounds strongly interact with MEK 2. The interaction prediction method according to the present embodiment predicts that the compounds more strongly interact with BRAF, IGF1R, Wee1, and the like.

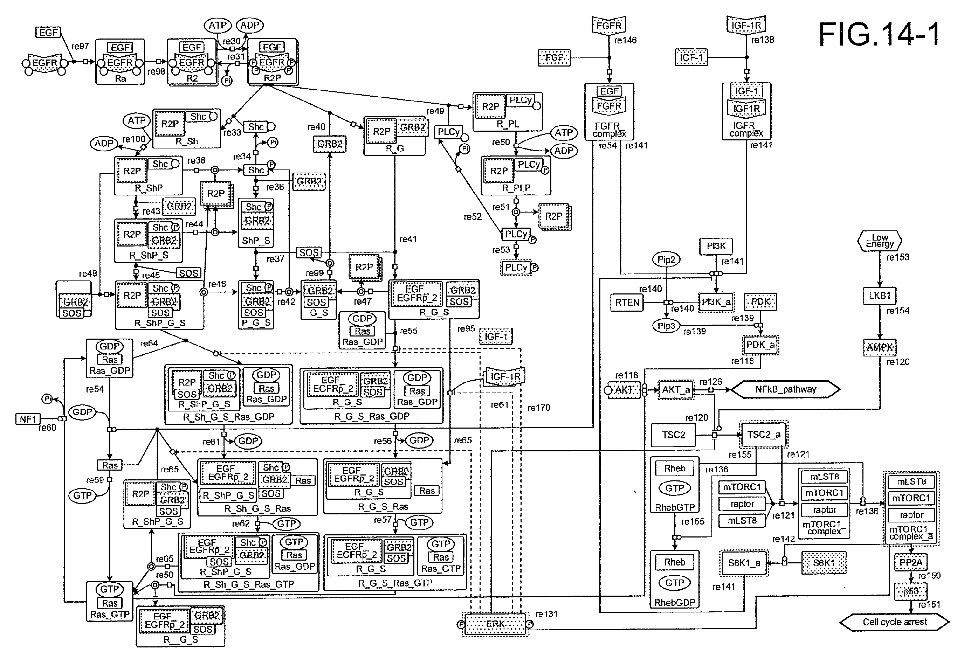

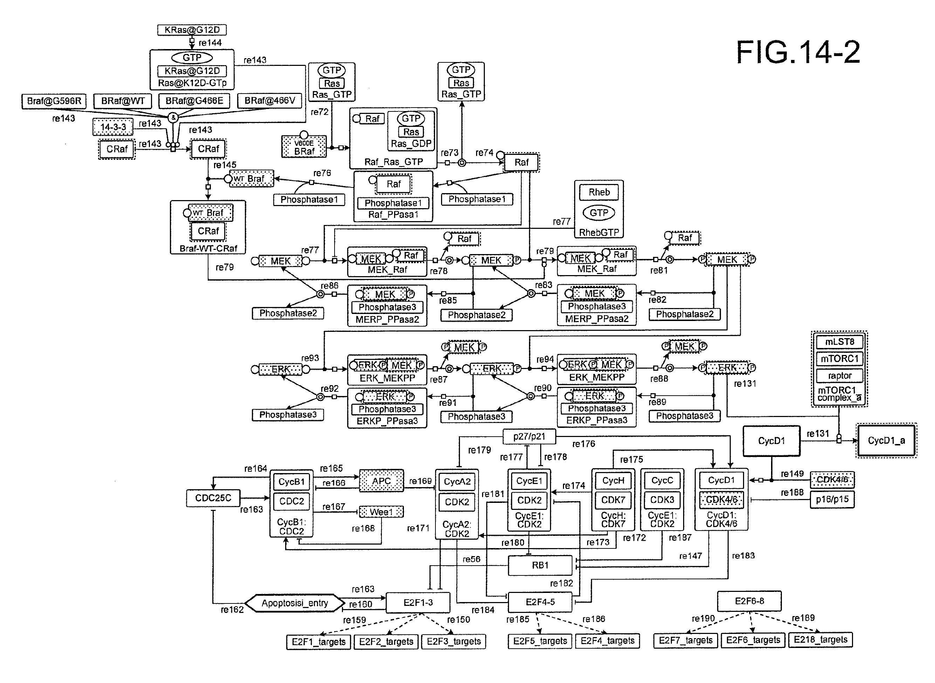

FIG. 14 is a schematic diagram obtained by color-coding an interaction network of biomolecules based on an interaction strength derived from the analysis result shown in FIG. 13. As shown in FIG. 14, coloring is performed on proteins predicted to interact with the compounds (AZD6244, CI-1040, PD0325901, and TAK-733) undergoing the clinical trial defining MEK as a target based on the analysis result shown in FIG. 13. These compounds interact with various types of proteins far beyond the expectations of the user (e.g., a pharmaceutical company). The interaction is distributed on a signal transduction system relating to the same biological function. It is doubtful whether the result obtained from cultured cells and the clinical trial is attributed to inhibition of the MEK protein. As shown in FIG. 14, the use of the interaction prediction method according to the present embodiment makes it more reasonable to assume that the compounds exert effects as a comprehensive result of interaction with BRAF, IGF1R, Wee1, APC, EGFR, IGF-1, and AKT1 besides inhibition of the MEK protein.

If each candidate compound interacts with each biomolecule in a predicted manner, the present embodiment needs to determine whether the candidate compound increases or decreases the activity of the biomolecule serving as the other side of the interaction. Also at this stage, the present embodiment employs a methodology for making a selection from a plurality of methods or a comprehensive determination. The present embodiment may use the meta-estimation system using the results obtained by a plurality of methods already used for the binding strength prediction. If the combination of the target biomolecule and the candidate compound is stored in a database on interaction between many biomolecules and compounds, information of activation and inactivation can be acquired from the data. If a known ligand or compound interacts with the target protein, for example, the present embodiment determines that the ligand or the compound activates the protein. If a target ligand or compound interacts with the protein in the same binding form, the compound is also assumed to activate the protein.

If a molecule that activates the protein competitively acts with the target ligand or compound, it is assumed that the ligand or the compound is inhibitory. Let us assume that a drug (compound) A binds to a specific binding region of a protein X and that a molecule Y simultaneously binding to the binding region of X activates the protein X, for example. In this case, the compound A and the molecule Y competitively interact with the same binding domain (binding pocket) of X. The drug A may possibly inhibit the interaction between the molecule Y and the protein X and function in an inhibitory manner. In this case, if the drug A and the molecule Y simply competitively interact with the same domain of the protein X, the drug A functions as an inhibitor of the interaction between the molecule Y and the protein X, making it uncertain whether the activation further promotes. To address this, if a database on molecules that interact with the same domain of the protein X and the action direction is available, the present embodiment may refer to the database.

In terms of major proteins, it is often experimentally known which portion of another protein each of the major proteins interacts with and what kind of effect the interaction results in. The present embodiment may use the database on the information, thereby estimating whether the candidate compound inhibits or activates the protein. If no such experimental data is known on which portion of another protein each of the major proteins interacts with and what kind of effect the interaction results in, but there is a combination of a biomolecule having a similar structure and the candidate compound, the present embodiment can determine activation or inactivation using the information. Every time a more precise method is developed, the present embodiment may update and newly introduce the new method. The similarity in the structure may be similarity in the whole molecule or a part (fragment) of the molecule. The present embodiment may also introduce a method for determining activation or inactivation based on a detailed position at which a biomolecule interacts with a candidate compound as long as the method is sufficiently accurate. The predictions made by these methods lead to a final result obtained by a method considering the characteristics of the methods. In this process, the present embodiment may introduce a method for making a final prediction using a method, such as a neural network and a statistical learning method. Thus, the present embodiment can derive a comprehensive influence of each candidate compound.

Let us assume that a calculation model is available in which parameters required to run a dynamic simulation by various types of method are already determined for a biomolecular interaction network relating to a target vital phenomenon. These parameters may be determined by making a calculation such that a behavior of the model coincides with that of experimental data with any one, some, or all of a genetic algorithm, stochastic annealing, and gradient descent using time-series data of a phosphorylated protein obtained by applying a known stimulus to a normal cell, for example.

The present embodiment may make a simulation calculation on what kind of change occurs when each candidate compound is applied to a biomolecule (protein) compared with a state where no candidate compound is applied to the biomolecule. In one method, the present embodiment may derive a behavior in a state where no candidate compound or the like is applied, thereby assuming a state where one candidate compound is applied. The present embodiment may set an equation with values of KD, Kd, Ka, and the like varying depending on the amount or the like of the candidate compound applied to each biomolecule predicted to interact with the candidate compound. The present embodiment can perform similar processing on a series of candidate compounds. At this stage, the calculation model can calculate how large difference in the intracellular response occurs when a certain amount of the candidate compound is applied to the biomolecule (protein) compared with a state where no candidate compound is applied.

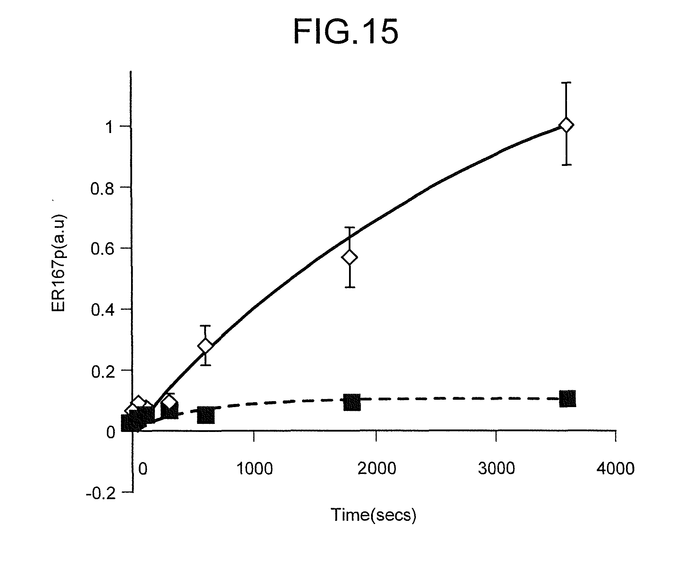

If a series of differential equations is set as a model of a signal transduction system of a cell, for example, an influence of the candidate compound is added to the differential equation. By solving the differential equation, responsiveness of the cell with the candidate compound applied is calculated and predicted. By making the calculation for the series of the candidate compounds, it is possible to predict what kind of effect each of the candidate compounds exerts on a target biological system. FIG. 15 is a graph of an example of calculation prediction (change prediction by a simulation calculation) according to the present embodiment. FIG. 15 is a graph indicating a computational prediction of a chronological change in the activity of the biomolecule (estrogen receptor) when a mutation occurs in a signal transduction system of a mammal cell. The solid line indicates a mutant type, whereas the dashed line indicates a normal type.

### Other embodiments

While the embodiment according to the present invention has been described, the present invention may be embodied in various different embodiments within the range of technical ideas described in the appended claims besides the embodiment described above.

An example where the interaction prediction device 100 performs processing in a stand-alone manner has been explained. The interaction prediction device 100 may perform processing in response to a request from a client terminal (a housing separated from the interaction prediction device 100) and transmit the processing result to the client terminal.

All or part of the processing explained to be automatically performed out of the processing explained in the embodiment may be manually performed. Alternatively, all or part of the processing explained to be manually performed may be automatically performed by a known method.

Furthermore, the processing procedures, the control procedures, the specific names, the information including the registration data of each processing and the parameters such as search criteria, the screen examples, and the database configurations indicated in the document and the drawings may be optionally changed unless otherwise provided.

The components of the interaction prediction device 100 shown in the drawings are functionally conceptual and are not necessarily physically configured as shown in the drawings.

All or desired part of the processing functions of each device in the interaction prediction device 100, particularly of the processing functions performed by the control unit 102 may be provided by a central processing unit (CPU) and a computer program interpreted and executed by the CPU or as wired logic hardware. The computer program is stored in a non-transitory computer-readable recording medium including a programmed instruction for causing a computer to perform the method according to the present invention, which will be described later. The computer program is mechanically read by the interaction prediction device 100 as needed. In other words, the storage unit 106, such as a ROM and a hard disk drive (HDD), stores a computer program for issuing an instruction to the CPU and performing various types of processing along with an operating system (OS). The computer program is loaded and executed on a RAM and serves as the control unit along with the CPU.

The computer program may be stored in an application program server connected to the interaction prediction device 100 via a desired network 300. The whole or part of the computer program may be downloaded as needed.

The computer program according to the present invention may be stored in a computer-readable recording medium or may be provided as a computer program product. Examples of the "recording medium" may include a desired "portable physical medium", such as a memory card, a USB memory, an SD card, a flexible disk, a magneto-optical disk, a ROM, an EPROM, an EEPROM, a CD-ROM, an MO, a DVD, and Blu-ray Disc.

The "computer program" is a data processing method described in a desired language and description method and is described in any format, such as a source code and a binary code. The "computer program" is not necessarily independently configured. The "computer program" may be configured dispersively as a plurality of modules and libraries or may carry out its function along with another computer program represented by the OS. In each device according to the embodiment, known configurations and procedures may be used for the specific configuration to read the recording medium, the reading procedure, the install procedure after the reading, or the like.

The various types of databases and the like stored in the storage unit 106 (the compound structure data database 106a, the protein structure data database 106b, the genetic data database 106c, the intermolecular interaction data database 106d, and the protein structure similarity data database 106e) correspond to a storage unit, such as a memory including a RAM and a ROM, a fixed disk drive including a hard disk, a flexible disk, and an optical disk. The databases store various types of computer programs, tables, databases, Web page files, and the like used for various types of processing and provision of websites.

The interaction prediction device 100 may be provided as an information processor, such as a known desktop or notebook personal computer, a mobile phone, a smartphone, a PHS, a portable terminal device including a PDA, and a workstation or as an information processor with desired auxiliary equipment. The interaction prediction device 100 may be provided by implementing software (including a computer program, data, and the like) for performing the method according to the present invention in the information processor.

The specific aspects of distribution and integration of the device are not limited to those shown in the drawings. All or a part of the components may be distributed or integrated functionally or physically in desired units depending on various types of additions and the like or functional loads. In other words, the embodiments above may be optionally combined or selectively provided.

### INDUSTRIAL APPLICABILITY

As explained above in detail, the present invention can provide an interaction prediction device, an interaction prediction method, and a computer program product that can predict which intravital protein a chemical substance, such as a compound, interacts with and how the interaction affects a living body. The present invention is extremely useful in various fields, such as medical care, drug development, drug discovery, and biological study.

### EXPLANATIONS OF LETTERS OR NUMERALS

- 100: interaction prediction device
- 102: control unit
- 102a: compound structure data acquiring unit
- 102b: protein structure data acquiring unit
- 102c: predicted protein determining unit
- 102d: interaction strength determining unit
- 102e: influence predicting unit
- 104: communication control interface
- 106: storage unit
- 106a: compound structure data database
- 106b: protein structure data database
- 106c: genetic data database
- 106d: intermolecular interaction data database
- 106e: protein structure similarity data database
- 108: input-output control interface
- 112: display unit
- 114: input unit
- 200: external system
- 300: network

## Claims

1. An interaction prediction device comprising:
a storage unit and a control unit, wherein
the storage unit includes:
a compound structure data storage unit that stores compound structure data on a structure of a compound; and
a protein structure data storage unit that stores protein structure data on a structure of a protein, and
the control unit includes:
a compound structure data acquiring unit that acquires the compound structure data on the compound from the compound structure data storage unit or predicts and acquires the compound structure data not stored in the compound structure data storage unit using a structure prediction method;
a protein structure data acquiring unit that acquires candidate protein structure data corresponding to the protein structure data on a candidate protein serving as the protein to be a candidate for interaction with the compound from the protein structure data storage unit or predicts and acquires the candidate protein structure data not stored in the protein structure data storage unit using the structure prediction method;
a predicted protein determining unit that calculates a binding strength between the candidate protein and the compound using a docking simulation method based on the compound structure data acquired by the compound structure data acquiring unit and the candidate protein structure data acquired by the protein structure data acquiring unit, determines a predicted binding strength corresponding to the binding strength eventually predicted by making a comprehensive evaluation of the binding strength using any one or both of a learning method and a meta-estimation method, and determines a predicted protein corresponding to the candidate protein predicted to interact with the compound; and
an interaction strength determining unit that calculates an interaction strength using a binding strength simulation method based on the compound structure data acquired by the compound structure data acquiring unit and the protein structure data on the predicted protein determined by the predicted protein determining unit and determines a predicted interaction strength corresponding to the interaction strength eventually predicted by making the comprehensive evaluation of the interaction strength using any one or both of the learning method and the meta-estimation method.

2. The interaction prediction device according to claim 1, wherein
the protein structure data storage unit stores the protein structure data on the structure of the protein in association with network data on an intracellular or intravital network including position data on the position of the protein on the network, and
the control unit further includes:
an influence predicting unit that predicts an influence of the compound on the predicted protein based on the predicted interaction strength determined by the interaction strength determining unit and the network data stored in the protein structure data storage unit.

3. The interaction prediction device according to claim 1 or 2, wherein
the storage unit further includes:
an intermolecular interaction data storage unit that stores intermolecular interaction data on intracellular or intravital intermolecular interaction, and
any one or both of the predicted protein determining unit and the interaction strength determining unit make the comprehensive evaluation further using the intermolecular interaction data stored in the intermolecular interaction data storage unit.

4. The interaction prediction device according to any one of claims 1 to 3, wherein
the storage unit further includes:
a protein structure similarity data storage unit that stores protein structure similarity data on similarity in the structure of the protein, and
any one or both of the predicted protein determining unit and the interaction strength determining unit make the comprehensive evaluation further using the protein structure similarity data stored in the protein structure similarity data storage unit.

5. The interaction prediction device according to any one of claims 1 to 4, wherein the protein structure data acquiring unit predicts and acquires the candidate protein structure data by predicting a plurality of pieces of protein structure data using the structure prediction method and making the comprehensive evaluation of the pieces of protein structure data using any one or both of the learning method and the meta-estimation method.

6. The interaction prediction device according to any one of claims 1 to 5, wherein
the storage unit further includes:
a genetic data storage unit that stores genetic data on a gene of an individual, and
the protein structure data acquiring unit predicts and acquires the candidate protein structure data using the structure prediction method based on the genetic data stored in the genetic data storage unit.

7. An interaction prediction method executed by an interaction prediction device including:
a storage unit and a control unit, wherein
the storage unit includes:
a compound structure data storage unit that stores compound structure data on a structure of a compound; and
a protein structure data storage unit that stores protein structure data on a structure of a protein,
the method executed by the control unit comprising:
a compound structure data acquiring step of acquiring the compound structure data on the compound from the compound structure data storage unit or predicting and acquiring the compound structure data not stored in the compound structure data storage unit using a structure prediction method;
a protein structure data acquiring step of acquiring candidate protein structure data corresponding to the protein structure data on a candidate protein serving as the protein to be a candidate for interaction with the compound from the protein structure data storage unit or predicting and acquiring the candidate protein structure data not stored in the protein structure data storage unit using the structure prediction method;
a predicted protein determining step of calculating a binding strength between the candidate protein and the compound using a docking simulation method based on the compound structure data acquired at the compound structure data acquiring step and the candidate protein structure data acquired at the protein structure data acquiring step, determining a predicted binding strength corresponding to the binding strength eventually predicted by making a comprehensive evaluation of the binding strength using any one or both of a learning method and a meta-estimation method, and determining a predicted protein corresponding to the candidate protein predicted to interact with the compound; and
an interaction strength determining step of calculating an interaction strength using a binding strength simulation method based on the compound structure data acquired at the compound structure data acquiring step and the protein structure data on the predicted protein determined at the predicted protein determining step and determining a predicted interaction strength corresponding to the interaction strength eventually predicted by making the comprehensive evaluation of the interaction strength using any one or both of the learning method and the meta-estimation method.

8. A computer program product having a non-transitory tangible computer-readable medium including programmed instructions for causing, when executed by an interaction prediction device including a storage unit including a compound structure data storage unit that stores compound structure data on a structure of a compound, and a protein structure data storage unit that stores protein structure data on a structure of a protein, and a control unit,
the control unit to perform a method comprising:
a compound structure data acquiring step of acquiring the compound structure data on the compound from the compound structure data storage unit or predicting and acquiring the compound structure data not stored in the compound structure data storage unit using a structure prediction method;
a protein structure data acquiring step of acquiring candidate protein structure data corresponding to the protein structure data on a candidate protein serving as the protein to be a candidate for interaction with the compound from the protein structure data storage unit or predicting and acquiring the candidate protein structure data not stored in the protein structure data storage unit using the structure prediction method;
a predicted protein determining step of calculating a binding strength between the candidate protein and the compound using a docking simulation method based on the compound structure data acquired at the compound structure data acquiring step and the candidate protein structure data acquired at the protein structure data acquiring step, determining a predicted binding strength corresponding to the binding strength eventually predicted by making a comprehensive evaluation of the binding strength using any one or both of a learning method and a meta-estimation method, and determining a predicted protein corresponding to the candidate protein predicted to interact with the compound; and
an interaction strength determining step of calculating an interaction strength using a binding strength simulation method based on the compound structure data acquired at the compound structure data acquiring step and the protein structure data on the predicted protein determined at the predicted protein determining step and determining a predicted interaction strength corresponding to the interaction strength eventually predicted by making the comprehensive evaluation of the interaction strength using any one or both of the learning method and the meta-estimation method.
